# EUROPÄISCHE PATENTSCHRIFT

(11) **EP 3 547 948 B1**
(45) Veröffentlichungstag und Bekanntmachung des Hinweises auf die Patenterteilung: **17.02.2021**
(21) Anmeldenummer: 17832200.4
(22) Anmeldetag: 30.11.2017
(51) Int. Cl.: A61C 5/00, A61C 17/16, A61C 17/02, A61C 1/00

(54) **VORRICHTUNG ZUM TROCKNEN VON ZAHN- ODER KNOCHENOBERFLÄCHEN**
DEVICE FOR DRYING TOOTH OR BONE SURFACES
DISPOSITIF DE SÉCHAGE DE SURFACES DENTAIRES OU OSSEUSES

(30) Priorität: 02.12.2016 DE 102016123345
(43) Veröffentlichungstag der Anmeldung: 09.10.2019
(73) Patentinhaber: Tilse, Rainer, 75172 Pforzheim (DE); Kraus, Tilman, 75173 Pforzheim (DE)
(72) Erfinder: Tilse, Rainer, 75172 Pforzheim (DE); Kraus, Tilman, 75173 Pforzheim (DE)
(74) Vertreter: Twelmeier Mommer & Partner
(86) Internationale Anmeldenummer: PCT/EP2017/080958
(87) Internationale Veröffentlichungsnummer: WO 2018/100056

(56) Entgegenhaltungen:
- EP-A2- 1 591 076
- WO-A1-00/09030
- WO-A1-2011/006793
- DE-A1- 10 049 068
- DE-A1-102007 002 593
- DE-A1-102012 107 589

## Beschreibung

Die Erfindung betrifft eine Vorrichtung zum Trocknen von Zahn- oder Knochenoberflächen.

Aus der EP 1 591 076 A1 ist eine Vorrichtung für dental-medizinische Behandlungen bekannt, die einen Laser zum Bestrahlen eines zu behandelnden Zahns und einen Temperatursensor enthält. Zudem ist es aus der WO 00/09030 A1 bekannt, Zahnoberflächen durch Bestrahlung mit einem Laser zu trocknen, und aus DE 10 2012 107 589 A1 eine Vorrichtung zur Trocknung von Wurzelkanälen mittels eines erwärmten Luftstroms bekannt.

Zur Behandlung von Karies wird betroffene Zahnsubstanz entfernt und dann durch Zahnfüllmasse ersetzt. Dabei ist wichtig, dass eine gute Haftung der Zahnfüllmasse auf der durch Entfernen von kariöser Zahnsubstanz gebildeten Zahnoberfläche erzielt wird. Damit dies möglich ist, muss die in der Regel nasse Zahnoberfläche zunächst getrocknet werden. Die Trocknung erfolgt dabei in der Regel, indem trockene Luft auf die Zahnoberfläche geblasen wird oder -wie in der WO 00/09030 A1 erwähnt- durch Bestrahlen.

In ähnlicher Weise müssen bei der chirurgischen Behandlung manchmal Oberflächen von Knochen getrocknet werden, beispielsweise beim Einsetzen von künstlichen Gelenken. Auch derartigen Fällen soll eine gute Klebeverbindung erzielt werden, nämlich zwischen dem Knochen einerseits und einem künstlichen Gelenk andererseits. Für eine gute Haftvermittlung darf dafür die Knochenoberfläche nicht nass sein, muss also getrocknet werden.

Aufgabe der vorliegenden Erfindung ist es, einen Weg aufzuzeigen, wie Zahnärzten bzw. Chirurgen die Trocknung von Zahn- oder Knochenoberflächen erleichtert werden kann, so dass eine optimale Klebeverbindung mit Zahnfüllmasse oder Haftvermittler erreicht werden kann.

Diese Aufgabe wird durch eine Vorrichtung mit den im Anspruch 1 angegebenen Merkmalen gelöst. Vorteilhafte Weiterbildungen der Erfindung sind Gegenstand von Unteransprüchen.

Eine erfindungsgemäße Vorrichtung enthält eine Strahlungsquelle, beispielsweise einen Laser oder eine Diode, um Strahlung zum Trocknen einer Zahn- oder Knochenoberfläche zu erzeugen, sowie einen Temperatursensor zum Messen der Temperatur der zu trocknenden Zahn- oder Knochenoberfläche und einen Feuchtigkeitssensor. Auf diese Weise kann eine Zahn- oder Knochenoberfläche durch Bestrahlen getrocknet und eine für eine gegebene Zahnfüllmasse bzw. einen gegebenen Haftvermittler optimale Feuchtigkeit erzielt werden. Mittels des Temperatursensors lässt sich dabei eine zu starke Erwärmung der Zahn- oder Knochenoberfläche, die zu Schäden führen könnte, verhindern. Der Feuchtigkeitssensor ermöglicht es, zu ermitteln, wann die Feuchtigkeit des Zahn- oder Knochenoberfläche in einem Bereich liegt, der für das Ausbilden einer guten Haftverbindung zu Zahnfüllmasse bzw. Knochenkleber oder Knochenzement optimal ist.

Insbesondere bei Zahnfüllmassen auf Kunststoffbasis ist nämlich die Feuchtigkeit der Zahnoberfläche für eine gute Haftung von entscheidender Bedeutung. Eine zu hohe Feuchtigkeit behindert das Ausbilden einer stabilen chemischen Verbindung zwischen Zahnsubstanz und Füllmaterial bzw. Haftvermittler. Eine völlige Trocknung bzw. zu geringe Feuchtigkeit führt aber ebenfalls nicht zu guten Ergebnissen, weil dann im Dentin enthaltene Kollagenfasern kollabieren und keine Bindung mehr mit dem Füllmaterial bzw. dem Haftvermittler eingehen. Sowohl zu nasse also auch zu trockene Zahnoberflächen führen also zu einer schlechten Klebeverbindung. Ähnlich ist es beim Verkleben von künstlichen Gelenken mit Knochen bei chirurgischen Anwendungen. Auch dabei darf für eine optimale Klebeverbindung die Knochenoberfläche nicht zu feucht und auch nicht zu trocken sein.

Eine vorteilhafte Weiterbildung der Erfindung sieht vor, dass der Feuchtigkeitssensor die Luftfeuchtigkeit misst, insbesondere die Luftfeuchtigkeit an der zu trocknenden Zahn- oder Knochenoberfläche. Die für die Haftung von Zahnfüllmasse oder Haftvermittler entscheidende Oberflächenfeuchtigkeit von Zahn oder Knochen korreliert stark mit der Luftfeuchtigkeit, insbesondere der Luftfeuchtigkeit nahe an der zu trocknenden Zahn- oder Knochenoberfläche. Aus einer Messung der Luftfeuchtigkeit lässt sich deshalb die Oberflächenfeuchtigkeit ermitteln. Dabei kann mittels empirischer Daten, die beispielsweise als Tabellen oder Kurven in einer Steuerung des Geräts gespeichert sind, aus der gemessenen Luftfeuchtigkeit die Oberflächenfeuchtigkeit der zu trocknenden Zahn- oder Knochenoberfläche bestimmt werden. Diese Genauigkeit dieser Bestimmung lässt sich erhöhen, wenn dabei auch die Temperatur der Zahn- oder Knochenoberfläche berücksichtigt wird, also beispielsweise die Oberflächenfeuchtigkeit mit einem Kennfeld als Funktion von Temperatur und Luftfeuchtigkeit bestimmt wird.

Der Feuchtigkeitssensor kann beispielsweise ein kapazitiver Feuchtigkeitssensor sein. Kapazitive Feuchtigkeitssensoren nutzen eine hygroskopische Schicht als Dielektrikum, in Regel aus Kunststoff oder Keramik, zwischen den beiden Elektroden eines Kondensators. Durch die Absorption von Feuchte im Dielektrikum ändern sich dessen Eigenschaften und in Folge die Kapazität des Kondensators. Der Feuchtigkeitssensor kann beispielsweise auch ein Impedanzsensor oder ein resistiver Sensor sein. Derartige Sensoren nutzen eine hygroskopische Schicht zwischen zwei Elektroden, deren elektrischer Widerstand sich durch die Absorption von Feuchte ändert.

Der Feuchtigkeitssensor kann auch photometrisch arbeiten, beispielsweise indem die Absorption von Infrarotlicht eines Lasers in einer definierten Wegstrecke gemessen wird. Die Absorption von Infrarotlicht in Luft erfolgt im Wesentlich durch Wasser, so dass sich mit dem Lambert-Beer'schen Gesetz die Luftfeuchtigkeit aus einer Absorptionsmessung berechnen lässt.

Der Temperatursensor kann beispielsweise ein berührungslos messendes Thermometer sein. Geeignet sind insbesondere Strahlungsthermometer, welche die Temperatur der zu trocknenden Zahn- oder Knochenoberfläche anhand der von der Zahn- oder Knochenoberfläche ausgesandten Wärmestrahlung ermitteln.

Eine weitere vorteilhafte Weiterbildung der Erfindung sieht vor, dass die Vorrichtung einen Abstandhalter zum Ansetzen an einen Zahn oder Knochen aufweist. Auf diese Weise lässt sich gewährleisten, dass der Feuchtigkeitssensor die Luftfeuchtigkeit in der Nähe der zu trocknenden Zahn- und Knochenoberfläche misst. Zudem kann der Abstandhalter die Luft an der zu trocknenden Zahn- oder Knochenoberfläche vor störenden Einflüssen, beispielsweise Atemluft, abschirmen. Der Abstandhalter kann dazu beispielsweise als ein Rohr ausgebildet sein. Der Feuchtigkeitssensor kann dann die Feuchtigkeit der Luft in dem Rohr weitgehend ohne Störeinflüsse messen. Die Strahlung zum Trocknen der Zahn- oder Knochenoberfläche fällt dann durch den von dem Rohr umgebenen Raum auf die zu trocknende Oberfläche.

Eine weitere vorteilhafte Weiterbildung der Erfindung sieht vor, dass die Vorrichtung eine Steuerung enthält, welche die Messsignale des Temperatursensors und des Feuchtigkeitssensors auswertet. Die Steuerung kann eine zu starke Erwärmung der zu trocknenden Zahn- oder Knochenoberfläche verhindern, indem die Strahlungsleistung entsprechend gesteuert wird. Eine Erwärmung der Zahn- oder Knochenoberfläche auf mehr als 42°C kann zu Gewebeschädigung führen und sollte deshalb vermieden werden. Aus Sicherheitsgründen kann aber auch eine niedrigere Obergrenze für die Temperatur der Zahn- oder Knochenoberfläche angesetzt werden, beispielsweise 41°C. Die Steuerung kann dabei die Temperatur der Zahn- oder Knochenoberfläche auf einen vorgegebenen Sollwert regeln, etwa auf einen Wert zwischen 39°C und 42°C.

Die Steuerung kann durch Auswertung der Signale des Temperatursensors und des Feuchtigkeitssensors ermitteln, wann die zu trocknende Zahn- oder Knochenoberfläche eine optimale Feuchtigkeit für das Auftragen von Zahnfüllmasse, Haftvermittler oder Ähnlichem aufweist, und dies einem Benutzer dann mittels einer Signalisierungseinheit anzeigen, beispielsweise durch ein optisches oder akustisches Signal. Auf diese Weise kann einem Benutzer also durch einen Signalton oder ein Signallicht anzeigt werden, dass der Trocknungsvorgang abgeschlossen ist.

Welche Oberflächenfeuchtigkeit für eine gute Klebeverbindung optimal ist, kann von den chemischen Besonderheiten der jeweiligen Zahnfüllmasse bzw. des Haftvermittlers abhängen. Für eine gegebene Zahnfüllmasse bzw. einen gegebenen Haftvermittler lässt sich jedoch stets die optimale Feuchtigkeit ermitteln, beispielsweise durch Laborversuche. Diese können in der Steuerung des Geräts vom Hersteller für eine empfohlene Zahnfüllmasse bzw. einen empfohlenen Haftvermittler eingespeichert werden. Möglich ist auch, dass ein Benutzer an der Vorrichtung einstellen kann, welche Feuchtigkeit durch den Trocknungsvorgang erreicht werden soll.

Weitere Einzelheiten und Vorteile der Erfindung werden an einem Ausführungsbeispiel der Erfindung unter Bezugnahme auf die beigefügte Zeichnung erläutert:

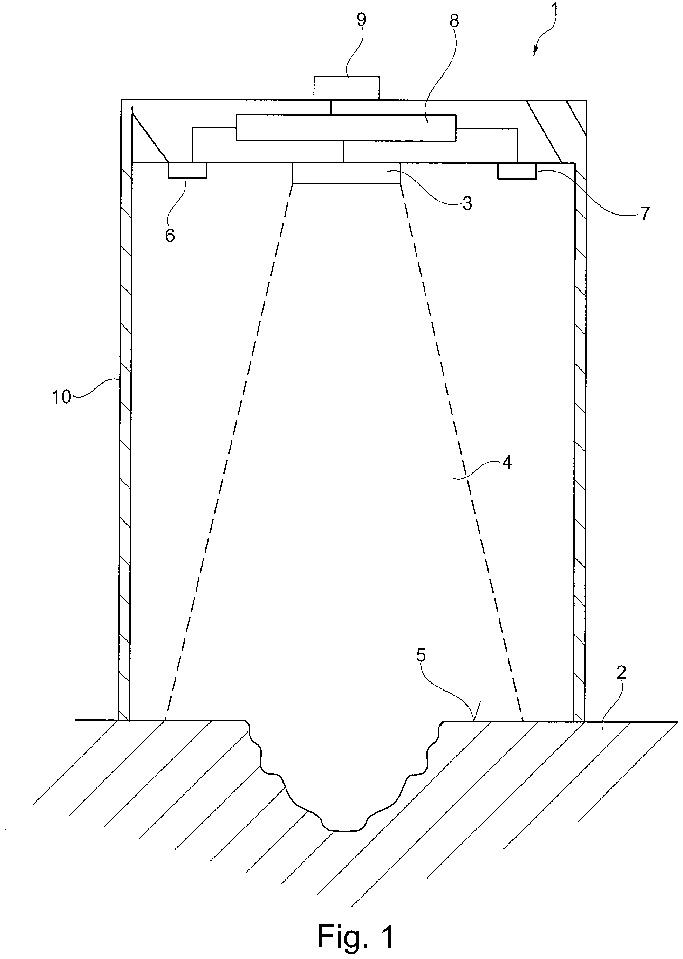

Fig. 1 zeigt eine schematische Darstellung einer Vorrichtung zum Trocknen von Zahn- oder Knochenoberflächen.

In Figur 1 ist schematisch in einer Schnittansicht eine Vorrichtung 1 zum Trocknen von Zahn- oder Knochenoberflächen zusammen mit einem Zahn 2 dargestellt. Die Vorrichtung 1 enthält eine Strahlungsquelle 3, beispielsweise ein Infrarotlaser oder eine Diode. Von der Strahlungsquelle 3 ausgesandte Strahlung 4, beispielsweise Infrarotstrahlung, trifft auf eine zu trocknende Zahn- oder Knochenoberfläche 5. Dies bewirkt, dass sich der Zahn oder Knochen erwärmt und Feuchtigkeit an der zu trocknenden Zahn- oder Knochenoberfläche 5 verdunstet.

Die Vorrichtung 1 enthält einen Temperatursensor 6 und einen Feuchtigkeitssensor 7, die ebenso wie die Strahlungsquelle 3 an eine Steuerung 8 angeschlossen sind. Der Temperatursensor 6 ist ein berührungslos messendes Thermometer und misst die Temperatur der zu trocknenden Zahn- oder Knochenoberfläche 5, beispielsweise indem Infrarotstrahlung erfasst und daraus die Oberflächentemperatur ermittelt wird. Der Feuchtigkeitssensor 7 misst die Luftfeuchtigkeit und kann beispielsweise ein kapazitiver oder resistiver Sensor sein.

Die Steuerung 8 wertet Messsignale des Temperatursensors 6 sowie des Feuchtigkeitssensors 7 aus und steuert die Strahlungsquelle 3. Die Steuerung 8 verhindert dabei, dass sich die Zahn- oder Knochenoberfläche 5 über eine vorgegebene Maximaltemperatur, von z.B. 42 °C, hinaus erwärmt, um eine Schädigung von Gewebe des Patienten zu verhindern. Die Steuerung 8 kann dabei die Temperatur der Zahn- und Knochenoberfläche 5 auf einen Sollwert regeln, der beispielsweise zwischen 39 °C und 41 °C liegt.

Die Steuerung 8 ermittelt durch Auswertung der Signale des Feuchtigkeitssensors 7 die Luftfeuchtigkeit. Aus der gemessenen Luftfeuchtigkeit und der Temperatur der Zahn- und Knochenoberfläche 5 lässt sich die Oberflächenfeuchtigkeit an der Zahn- oder Knochenoberfläche 5 ermitteln, beispielsweise mittels empirischer Daten, die in der Steuerung 8 als Tabellen, Kennlinien oder Kennfelder gespeichert sein können. Sobald die Steuerung 8 eine Feuchtigkeit feststellt, die in einem für die betreffende Zahnfüllmasse bzw. den betreffenden Haftvermittler eine optimale Bindung ermöglicht, beendet die Steuerung 8 den Trocknungsvorgang automatisch und signalisiert dies durch Betätigen der an die Steuerung 8 angeschlossenen Signalisierungseinheit 9, die beispielsweise ein akustisches oder optisches Signal erzeugt.

Damit die gemessene Luftfeuchtigkeit möglichst wenig durch Störeinflüsse, beispielsweise feuchte Atemluft, beeinträchtigt wird, kann die Vorrichtung einen Abstandhalter 10 in Form eines Rohres aufweisen. Der Abstandhalter 10 umgibt den von der Strahlungsquelle 3 ausgesandten Strahlungskegel 4 und den Feuchtigkeitssensor 7 sowie den Temperatursensor 6.

### Bezugszeichenliste

- 1: Vorrichtung
- 2: Zahn
- 3: Strahlungsquelle
- 4: Strahlungskegel
- 5: Zahn- oder Knochenoberfläche
- 6: Temperatursensor
- 7: Feuchtigkeitssensor
- 8: Steuerung
- 9: Signalisierungseinheit
- 10: Abstandhalter

## Patentansprüche

1. Vorrichtung zum Trocknen von Zahn- oder Knochenoberflächen (5), mit
einer Strahlungsquelle (3) zum Bestrahlen der zu trocknenden Zahn- oder Knochenoberfläche (5), und
einem Temperatursensor (6), **gekennzeichnet durch**
einen Feuchtigkeitssensor (7).

2. Vorrichtung nach Anspruch 1, **dadurch gekennzeichnet, dass** der Feuchtigkeitssensor (7) ein Sensor zur Bestimmung der Luftfeuchtigkeit ist.

3. Vorrichtung nach einem der vorstehenden Ansprüche, **dadurch gekennzeichnet, dass** die Strahlungsquelle (3) ein Laser, vorzugsweise ein Infrarotlaser ist.

4. Vorrichtung nach einem der vorstehenden Ansprüche, **gekennzeichnet durch** einen Abstandhalter (10) zum Ansetzen an einen Zahn (2) oder Knochen aufweist.

5. Vorrichtung nach einem der vorstehenden Ansprüche, **dadurch gekennzeichnet, dass** der Abstandhalter (10) als ein Rohr ausgebildet ist.

6. Vorrichtung nach einem der vorstehenden Ansprüche, **gekennzeichnet durch** eine Steuerung (8), welche die Strahlungsquelle (3) in Abhängigkeit von Signalen des Temperatursensors (6) und des Feuchtigkeitssensors (7) steuert.

7. Vorrichtung nach Anspruch 6, **dadurch gekennzeichnet, dass** die Steuerung (8) die Strahlungsquelle (3) so steuert, dass eine vorgegebene Maximaltemperatur der zu trocknenden Zahn- oder Knochenoberfläche (5) nicht überschritten wird.

8. Vorrichtung nach Anspruch 6 oder 7, **dadurch gekennzeichnet, dass** die Steuerung (8) durch Steuern der Strahlungsquelle (3) die Temperatur der zu trocknenden Zahn- oder Knochenoberfläche (5) auf einen Sollwert regelt.

9. Vorrichtung nach einem der vorstehenden Ansprüche, **dadurch gekennzeichnet, dass** der Temperatursensor (8) ein Strahlungssensor zur berührungslosen Temperaturmessung der zu trocknenden Zahn- oder Knochenoberfläche (5) ist.

10. Vorrichtung nach einem der vorstehenden Ansprüche, **gekennzeichnet durch** eine Signalisierungseinheit (9), die ein Signal erzeugt, sobald der Feuchtigkeitssensor (7) eine Feuchtigkeit ermittelt, die einen vorgegebenen Schwellenwert erreicht oder unterschreitet.

## Claims

1. A device for the drying of tooth or bone surfaces (5), comprising
a radiation source (3) for irradiating the tooth or bone surface (5) to be dried, and a temperature sensor (6), **characterized by**
a humidity sensor (7).

2. The device in accordance with claim 1, **characterised in that** the humidity sensor (7) is a sensor for determining air humidity.

3. The device in accordance with anyone of the preceding claims, **characterised in that** the radiation source (3) is a laser, preferably an infrared laser.

4. The device in accordance with anyone of the preceding claims, **characterised by** a spacer (10) to be set in position on a tooth (2) or bone.

5. The device in accordance with anyone of the preceding claims, **characterised in that** the spacer (10) is designed as a tube.

6. The device in accordance with anyone of the preceding claims, **characterised by** a controller (8), which controls the radiation source (3) as a function of signals from the temperature sensor (6) and the humidity sensor (7).

7. The device in accordance with claim 6, **characterised in that** the controller (8) controls the radiation source (3) such that a predetermined maximum temperature of the tooth or bone surface (5) to be dried is not exceeded.

8. The device in accordance with claims 6 or 7, **characterised in that** the controller (8) controls the temperature of the tooth or bone surface (5) to be dried to a setpoint value by controlling the radiation source (3).

9. The device in accordance with anyone of the preceding claims, **characterised in that** the temperature sensor (8) is a radiation sensor for non-contacting temperature measurement of the tooth or bone surface (5) to be dried.

10. The device in accordance with anyone of the preceding claims, **characterised by** a signaling unit (9), which generates a signal as soon as the humidity sensor (7) detects a humidity that reaches or falls below a predetermined threshold value.

## Revendications

1. Dispositif de séchage de surfaces dentaires ou osseuses (5), doté d'une source de rayonnement (3) permettant d'éclairer la surface dentaire ou osseuse (5) devant être séchée, et
un capteur de température (6), **caractérisé par**
un capteur d'humidité (7).

2. Dispositif selon la revendication 1, **caractérisé en ce que** le capteur d'humidité (7) est un capteur de détermination de l'humidité de l'air.

3. Dispositif selon l'une des revendications précédentes, **caractérisé en ce que** la source de rayonnement (3) est un laser, de préférence un laser infrarouge.

4. Dispositif selon l'une des revendications précédentes, **caractérisé par**, présente, un espaceur (10) pour être placé sur une dent (2) ou un os.

5. Dispositif selon l'une des revendications précédentes, **caractérisé en ce que** l'espaceur (10) est conçu sous forme d'un tube.

6. Dispositif selon l'une des revendications précédentes, **caractérisé par** un système de commande (8), lequel commande la source de rayonnement (3) en fonction de signaux du capteur de température (6) et du capteur d'humidité (7).

7. Dispositif selon la revendication 6, **caractérisé en ce que** le système de commande (8) commande la source de rayonnement (3) de telle manière qu'une température maximale prédéfinie de la surface dentaire ou osseuse (5) à sécher n'est pas dépassée.

8. Dispositif selon la revendication 6 ou la revendication 7, **caractérisé en ce que** le système de commande (8) règle la température de la surface dentaire ou osseuse (5) à sécher sur une valeur prescrite.

9. Dispositif selon l'une des revendications précédentes, **caractérisé en ce que** le capteur de température (8) est un capteur de rayonnement pour la mesure de la température sans contact de la surface dentaire ou osseuse (5) à sécher.

10. Dispositif selon l'une des revendications précédentes, **caractérisé par** une unité de signalisation (9) qui génère un signal dès que le capteur d'humidité (7) détermine une humidité qui atteint une valeur limite prédéfinie ou y est inférieure.
